# EUROPEAN PATENT SPECIFICATION

(11) **EP 2 831 025 B1**
(45) Date of publication and mention of the grant of the patent: **23.08.2017**
(21) Application number: 13712745.2
(22) Date of filing: 25.03.2013
(51) Int. Cl.: C07C 31/04, C07C 29/151

(54) **CONTINUOUS PROCESS FOR THE PREPARATION OF METHANOL BY HYDROGENATION OF CARBON DIOXIDE**
DURCHGEHENDES VERFAHREN ZUR HERSTELLUNG VON METHANOL DURCH HYDRIERUNG VON KOHLENDIOXID
PROCÉDÉ CONTINU POUR LA PRÉPARATION DE MÉTHANOL PAR HYDROGÉNATION DU DIOXYDE DE CARBONE

(30) Priority: 28.03.2012 EP 12161706; 28.03.2012 US 201261616510 P
(43) Date of publication of application: 04.02.2015
(73) Proprietor: Akzo Nobel Chemicals International B.V., 6824 BM Arnhem (NL)
(72) Inventor: KISS, Anton Alexandru, NL-6835 CM Arnhem (NL); PRAGT, Johannes Jozef, NL-6951 MG Dieren (NL); VAN IERSEL, Maikel Maria, NL-5223 MP Den Bosch (NL); BARGEMAN, Gerrald, NL-6708 NW Wageningen (NL); DE GROOT, Matheus Theodorus, NL-3581 RN Utrecht (NL)
(74) Representative: Akzo Nobel IP Department
(86) International application number: PCT/EP2013/056175
(87) International publication number: WO 2013/144041

(56) References cited:
- WO-A1-2007/108014
- GB-A- 1 189 017

## Description

The present invention relates to a continuous process for the preparation of methanol.

Methanol is typically produced from syngas. Syngas is the name given to a gas mixture that mainly consists of carbon monoxide and hydrogen. Syngas is most commonly produced by steam reforming of natural gas:

CH₄ + H₂O → CO + 3 H₂

In a subsequent reaction, the carbon monoxide and hydrogen react in the presence of a catalyst to form methanol.

CO + 2 H₂ → CH₃OH

Today, the most widely used catalyst in the conversion of syngas is a mixture of copper, zinc oxide, and alumina.

Steam reforming of natural gas produces hydrogen in excess of ideal stoichiometric quantities for the methanol synthesis reaction and often carbon dioxide is added to improve the hydrogen-to-carbon ratio. Alternatively, methanol can also be produced directly from carbon dioxide and hydrogen without the intermediate production of syngas, allowing the use of an abundant carbon feedstock for the production of methanol:
1)

   CO₂ + 3H₂ ⇆ CH₃OH + H₂O
2)

   CO₂ + H₂ ⇆ CO + H₂O
3)

   CO + 2H₂ ⇆ CH₃OH

In the search for non-fossil based production routes to methanol the focus has mainly been on biomass gasification to produce syngas and the subsequent conversion to methanol. The direct route starting from carbon dioxide and hydrogen is preferred over this route, as it avoids the energy- and capital-intensive intermediate production of syngas. Additionally, the possibility to introduce both reactants as separate streams has the potential to further improve resource efficiency, in line with the desire for more sustainable production routes.

A process wherein methanol is produced from carbon dioxide (CO₂) and hydrogen is known for example from WO 2007/108014. In this document an example is provided wherein H₂ and CO₂ are supplied at 50 bar pressure and a molar ratio of 3:1 (H₂ to CO₂) to a methanol synthesis reactor loaded with a Cu/ZnO/Al₂O₃, Cu/ZrO₂ or Cu-Zn-Cr based catalyst. The reactor is operated at 225°C and 20% of the input stream reacts to methanol and water in an equal ratio. After exiting the reactor, the output stream is further cooled so as to condense the reaction products (water and methanol). The condensed products are collected and periodically ejected, while the non-condensed gases are recirculated by a circulation pump and combined with fresh feed stream. Methanol is recovered using a conventional separation process as used in the classical, syngas-based route to methanol. This implies that any carbon dioxide that remains dissolved in the liquid product stream after a condensation step is lost in the separated liquid phase, which is a major disadvantage in terms of resource efficiency. Furthermore, the crude methanol obtained via this process comprises carbon monoxide (CO). Purifying a CO-comprising methanol stream is undesired due to safety issues.

It is therefore an object of the present invention to provide a process with improved resource efficiency and lower energy consumption than the currently known processes to produce methanol from CO₂ and H₂. It is furthermore an object of the present invention to provide a safer process for the production of methanol than the one disclosed in WO 2007/108014, while at the same time improving the methanol yield based on CO₂.

The objectives have been met by providing a continuous process for the preparation of methanol wherein a carbon dioxide feed and a hydrogen feed are fed to the process separately from each other, wherein the produced methanol-comprising product stream is stripped using a hydrogen stream to remove remaining CO₂ and CO, and wherein at least part of the produced streams comprising CO₂, CO, and hydrogen is recycled to said reactor. More particularly, by using hydrogen gas stripping the majority if not all of the carbon dioxide reactant is recovered and recycled back to the reactor hence allowing a complete (i.e. exceeding 99.8%) overall conversion of carbon dioxide as well as hydrogen. This leads to a process with improved resource efficiency and lower energy consumption than the currently known processes to produce methanol from CO₂ and H₂. Furthermore, by the stripping step according to the present invention, the amount of CO in the methanol-comprising product stream is significantly reduced if not removed completely. The obtained crude methanol can thus be purified, e.g. by distillation, in a safe manner.

More particularly, the present invention relates to a continuous process for the preparation of methanol by hydrogenation of carbon dioxide in the presence of a solid catalyst comprising the steps of
(i) feeding
   - (a) a feed comprising carbon dioxide;
   - optionally, (b) a hydrogen feed,
   - (c) at least part of a first recycle gas stream comprising carbon dioxide and hydrogen; and
   - optionally, (d) at least part of a second recycle gas stream comprising carbon dioxide, carbon monoxide, and hydrogen,
   to a reactor, to obtain a gaseous feed with a hydrogen : carbon dioxide molar ratio of between 2-18 : 1;
(ii) in said reactor, contacting said gaseous feed with a catalyst at a temperature of between 200 and 300°C and a pressure of between 40 and 200 bar, thereby forming an outlet stream comprising methanol, water, carbon monoxide, carbon dioxide, and hydrogen;
(iii) cooling the outlet stream;
(iv) subjecting said cooled outlet stream to a separation step, while optionally at least part of a second recycle gas stream is added to said outlet stream comprising methanol prior to and/or during said separation step, in which separation step methanol and water are separated from non-condensable components, thereby forming a methanol-comprising product stream and a first recycle gas stream;
(v) stripping the methanol-comprising product stream using a hydrogen stream, thereby forming a purified methanol product stream and a second recycle gas stream comprising carbon dioxide, carbon monoxide, and hydrogen; and
(vi) feeding at least part of the first recycle gas stream to step (i) and at least part of the second recycle gas stream to steps (i) and/or (iv).

Preferably, the amount of CO₂ in the first recycle stream is between 55 and 90 wt%, based on the total weight of said first recycle stream. Preferably, the amount of hydrogen is between 4 and 40 wt%, based on the total weight of said first recycle stream. The amounts of all components present in the first recycle stream will together add up to 100 wt%.
Preferably, the amount of carbon dioxide in the second recycle gas stream is between 5 and 70 wt%, based on the total weight of said second recycle stream. The amount of carbon monoxide is preferably between 0.01 and 2 wt%, based on the total weight of said second recycle stream, and the amount of hydrogen is preferably between 25 and 85 wt%, based on the total weight of said second recycle stream. The amount of methanol in the second recycle gas stream is preferably between 0.1 and 10 wt%, based on the total weight of said second recycle stream. The amounts of all components present in the second recycle stream will together add up to 100 wt%.

Preferably, the gaseous feed of step (i) has a H₂ : CO₂ molar ratio of between 2.5-7 : 1. Hydrogen in excess of the ideal stoichiometric quantity is used to improve the single-pass conversion. Higher ratios are not preferred as this increases the total recycle gas flow, rendering the process less economical. In one embodiment, the feed comprising carbon dioxide, optionally part of the hydrogen feed, the first recycle stream comprising carbon dioxide and hydrogen, and the second recycle stream comprising carbon dioxide, carbon monoxide and hydrogen, are fed to the reactor as one combined gaseous feed. However, it is also possible to feed (part of) one or more of these streams to the reactor via another inlet. In any case, the streams are fed to the reactor in such amounts that in the reactor, a gaseous feed is obtained having a H₂ : CO₂ molar ratio of between 2-18 : 1, preferably a H₂ : CO₂ molar ratio of between 2.5-7 : 1. For the sake of clarity, it is possible to introduce hydrogen to the process merely via step (v) and not via step (i). Preferably, at least 20 wt% of the total amount of hydrogen needed in the process in order to obtain the required H₂ : CO₂ ratio will be fed to the process via step (v).

The term "solid catalyst" as used throughout this specification is meant to denote any catalyst for the synthesis of methanol from CO₂, hydrogen, and optionally CO which will remain in the reactor and not be removed together with the outlet stream. For the sake of clarity, this means that the solid catalyst according to the present invention also includes immobilized catalysts for the synthesis of methanol from CO₂, hydrogen, and optionally CO (i.e. immobilized liquid phase catalysts).

Preferably, the reactor to which a feed comprising carbon dioxide, a first recycle gas stream comprising carbon dioxide and hydrogen, and optionally a second recycle gas stream comprising carbon dioxide, hydrogen, and carbon monoxide, and optionally a hydrogen feed are fed (in step (i) of the process according to the present invention) is a reactor selected from the group consisting of a multi-tubular plug-flow reactor, a fixed bed reactor, and a fluidized bed reactor. These types of reactors are all commonly known in the art. Optionally, this reactor contains separate catalyst beds (stages) with interstage gas redistribution and possibly fresh gas supply.
The combined gaseous stream obtained in step (i) is preferably heated in a feed-effluent heat-exchanger (FEHE) by the outlet stream obtained in step (ii) prior to being subjected to step (ii), *i.e.* prior to being contacted with a catalyst at a temperature of between 200 and 300°C and a pressure of between 40 and 200 bar. Preferably, the temperature is at least 220°C, more preferably at least 240°C. Preferably, the temperature is below 280°C, more preferably, the temperature is below 260°C. Preferably, the pressure is at least 45 bar, but below 150 bar, more preferably below 100 bar. Most preferably, the pressure is around 50 bar.

In another embodiment according to the present invention, in step (i) at least part of the gaseous feed is fed along the reactor at different stages to improve removal of reaction heat and to reduce pressure drop over the reactor. More preferably, between 10-90 vol% of the gaseous feed is fed to the reactor via a reactor inlet at the top of the reactor, between 10-90 vol% of said gaseous feed is fed to the reactor via a reactor inlet at between 1/8 and 1/5 of reactor length, specified from the top of said reactor, and between 10-90 vol% of said gaseous feed is fed to the reactor via a reactor inlet at between 1/4 and 1/2 of reactor length, specified from the top of said reactor. The gaseous feed that is fed along the reactor at different stages can be at least part of the feed comprising carbon dioxide, at least part of the hydrogen feed, at least part of the first recycle gas stream, at least part of the second recycle gas stream, or any combination thereof. In a preferred embodiment, at least part of a combined feed comprising (a) the feed comprising carbon dioxide, optionally (b) the hydrogen feed, (c) the first recycle gas stream, and optionally (d) the second recycle gas stream, is fed along the reactor at different stages.

As a skilled person will understand, the hydrogen stream of step (v) and/or the hydrogen feed of step (i) may comprise limited amounts of other components such as methanol, CO₂, CO and/or water.
In another embodiment of the present invention, the hydrogen stream of step (v) and/or the hydrogen feed of step (i) preferably are fresh hydrogen streams. The term "fresh hydrogen stream" is meant to include any source of hydrogen gas, with the exception of a recycle hydrogen stream from the process according to the present invention. The fresh hydrogen stream used is selected from the group consisting of hydrogen produced by steam reforming of natural gas, dissociation of hydrocarbons, and electrolysis.
Most preferably, the hydrogen stream of step (v) and/or the hydrogen feed of step (i) are hydrogen from a wet hydrogen source. By the term "wet hydrogen source" is meant a hydrogen source which was produced in an aqueous electrolysis process, e.g. in the production of chlorine or sodium chlorate (i.e. a hydrogen source obtained from a chlorine production plant or a sodium chlorate production plant). Surprisingly, it was found that wet hydrogen can be used in the process according to the present invention, as it was found that no additional drying step is required if said wet hydrogen is fed to the process via step (v). The water is removed in the stripping step. It is economically beneficial to use a wet hydrogen source as this hydrogen source is a cheaper hydrogen source than dry hydrogen. A wet hydrogen source which can be used in the process according to the present invention typically comprises between 0.1 and 5 vol.% of water, more preferably between 1 and 2 vol.% of water.

If a wet hydrogen source is used, it is preferred to introduce the majority thereof to the process via step (v) instead of step (i).

The carbon dioxide feed suitable for use in the process according to the present invention can be any carbon dioxide feed known in the art. In one embodiment of the present invention, the carbon dioxide feed is carbon dioxide obtained from a bioethanol fermentation process. Preferably, the source of carbon dioxide is already pressurized, such as is for instance available from carbon capture and storage activities.

The catalyst suitable for use in the process according to the present invention can be any catalyst known in the art for methanol synthesis from syngas or any other CO₂ and/or CO and H₂-comprising reaction mixtures. Preferably, it is a Cu/ZnO-based catalyst. More preferably, it is a Cu/ZnO/Al₃O₂/ZrO₂ catalyst (as for example described in X. An et al., "Catalysis, Kinetics and Reactors," Chinese Journal of Chemical Engineering, 17(1), 88-94 (2000)) or a Cu/ZnO/ZrO₂/Al₂O₃SiO₂ catalyst (as for example described in J. Toyir et al., Physics Procedia 2 (2009)). Other catalysts that are suitable for use in the process according to the present invention are for instance the catalysts as described in EP 794006, EP 868943, US 6376562, and EP 721799. The Katalco™ catalysts series of Johnson Matthey for the synthesis of methanol from CO, CO₂, and hydrogen is also suitable for use in the process according to the present invention.

Preferably, step (iii) wherein the outlet stream is cooled to separate the condensable components (methanol and water) is carried out in a feed-effluent heat-exchanger (FEHE) and subsequently in a cooler, or, optionally, in a reboiler of a stripping column or distillation column in order to cool down by providing heat to the reboiler of the stripping column or the distillation column. The temperature is preferably lower than the saturation temperature of the methanol-water mixture at the specified pressure to make sure that methanol and water will condense.

As described above, in step (iv), formed methanol and water are separated from non-condensable components, thereby forming a methanol-comprising product stream and a first recycle gas stream. The term "non-condensable components" is meant to denote all components that will not condense at the specified pressure range. Non-condensable components in the present process include CO, CO₂, and hydrogen.
Optionally, part of the first recycle gas stream is purged. As the skilled person will understand, whether or not this is preferred depends on the purity of the feed.
It is possible, and even preferred, to recycle at least part of the second recycle gas stream (i.e. the gas stream comprising carbon dioxide, carbon monoxide, and hydrogen, obtained from the stripping step (v)) to step (iv). The second recycle gas stream can be added in part or completely to the outlet stream of the reactor prior to the separation step. It can also be added in part or completely to the separation step (iv). However, it is also possible to add said gas stream, in part or completely, during the separation step. Obviously, a combination of these two is also possible.

In step (v), the methanol-comprising product stream is stripped using a hydrogen stream, thereby forming a purified methanol product stream and a second recycle gas stream comprising carbon dioxide, hydrogen, and carbon monoxide. Said step is preferably performed in a stripping column with trays or packing internals or in a flash vessel. More preferably, the stripping column comprises a partial condenser. The partial condenser separates the gas phase leaving the top of the stripping column into a condensate stream which is recycled to the stripping column, and a stream which is denoted as second recycle gas stream throughout the specification.

In step (vi), at least part of the first recycle gas stream and at least part of the second recycle gas stream are recycled to step (i).

Preferably, the process according to the present invention comprises a step (vii), wherein the methanol-comprising product stream is concentrated and/or purified. This is preferably done by distillation, most preferably using a dividing wall column. The methanol-comprising product stream comprises water and possibly other impurities. For most applications of methanol it is necessary to remove part if not all of the water. This can be achieved by any method known to the person skilled in the art, for example distillation, for instance using a dividing wall column.

In another embodiment of the present invention, step (ii) of the process is carried out in two reactors instead of in one, with intermediate removal of water formed in the first reactor (similar to the principle disclosed by O.-S. Joo et al. in Ind. Eng. Res. 1999, 38, pp. 1808-1812). In this way, a higher carbon dioxide and hydrogen conversion is obtained in step (ii), compared to the embodiment wherein one reactor is used. This higher conversion means that a smaller amount of hydrogen, carbon dioxide, and carbon monoxide needs to be recycled in the process, which results in lower energy and equipment costs related to this recycle. Furthermore, two separate water-methanol streams are formed that have different concentrations. By feeding these streams into the distillation column at appropriate locations - in stages with similar compositions compared to the feed stream - it is possible to separate the methanol from the water in these streams in a way that costs less energy than if only one water-methanol stream was formed. Finally, a lower water level is obtained in the second reactor, which results in increased stability of the catalyst in this reactor. This is related to the fact that catalyst activity is decreased by the presence of water.
In this embodiment, the first reactor is preferably operated at a relatively high temperature of at least 250°C, preferably of at least 270°C, but preferably not higher than 350°C, more preferably not higher than 285°C, as this favours the reverse water gas shift reaction (reaction 2, *vide supra*) relative to the other reactions. The other two reactions, however, still occur, resulting in an overall small heat generation in the first reactor, which is operated adiabatically. After the first reactor, most of the formed water and part of the formed methanol are removed. This can be achieved in any conventional way, for example via a condensing step. The remaining mixture consisting of mostly CO and hydrogen is subsequently fed to a second reactor. This reactor is operated at a lower temperature than the first reactor, preferably between 220°C and 260°C, more preferably between 235°C and 245°C, so that the exothermic methanol forming reactions are favoured (reactions 1 and 3, *vide supra*). The heat generated in this reactor is preferably used to generate steam that can be further used in for example the separation section of the process. The formed methanol is subsequently cooled and separated after the second reactor according to steps (iii) and (iv), respectively, of the process according to the present invention.

As mentioned above, the intermediate water removal step is preferably performed in a conventional separator. In this step a liquid stream is obtained comprising water (typically between 60 - 65 wt%), methanol (typically between 30 and 40 wt%), and CO₂ (typically between 0.2 and 1 wt%), and a third gas recycle stream is obtained. Preferably, the third gas recycle stream is fed to the second reactor. The first and second recycle streams can also be fed to this second reactor.

The present invention is further illustrated by the following non-limiting Examples.

### Example 1

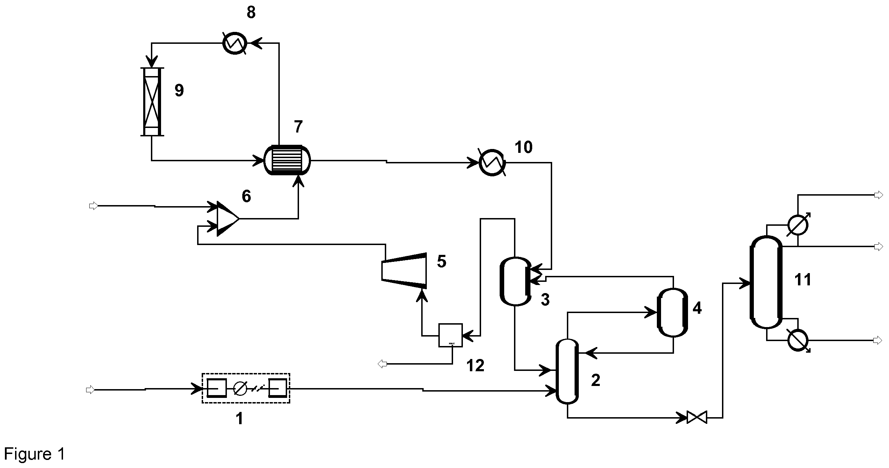

A rigorous process model was built in AspenTech Aspen Plus, using a PFR model for the reactor and a RADFRAC model for the separation units (stripping and distillation columns). Soave-Redlich-Kwong EOS and NRTL with Henry components were used as the most adequate property models.
A flow sheet of this process is depicted in Figure 1.

In the process used for the calculations, a wet hydrogen feed stream (2,460 kg/hr, 35°C, and 1.1 bar, containing 1.4 %vol. water) was compressed via a multi-stage compressor **1** (to 45 bar and 170°C) and then fed to the bottom of a stripping column **2** with 4 theoretical stages (operated at 50 °C). The counter-current feed stream fed to the top of the stripping column **2** was the methanol and water-rich liquid stream (18,755 kg/hr, 62.6 %wt MeOH, 34.7 %wt H₂O, 2.6 %wt CO₂, and 53 ppm CO, i.e. the methanol-comprising product stream) coming from the flash separation unit **3**.
The top outlet of the stripping column **2** was cooled in a stripping column partial condenser **4** at 30°C and 45 bar and separated into a liquid stream and a gas stream (i.e. the second recycle gas stream). The liquid stream was returned to the stripping column **2**, whereas the gas stream was subsequently sent back to the flash separation unit **3**, and consists of H₂ (77.1 %wt), CO₂ (17.1 %wt), MeOH (5.8 %wt), CO (350 ppm), and water (130 ppm). The gas has a molar ratio H₂O:H₂ = 1.89·10⁻⁵ which is much lower than in the wet hydrogen feed (H₂O:H₂ = 1.38·10⁻²).
The top gas outlet of the stripping column partial condenser **4** (containing CO₂, CO, and 98.5 %vol H₂, being the second recycle gas stream) was fed, together with the cooled reactor outlet stream comprising H₂ (8.7 %wt), CO₂ (68 %wt), MeOH (11.4 %wt), CO (6.1 %wt), and water (5.8 %wt), to the flash separation unit **3** operated at 45 bar and 30°C. The liquid outlet of the flash separation unit **3** was fed to the top of the stripping column **2** (as previously mentioned), while the vapour/gas outlet (i.e. the first recycle gas stream) was fed to a recycle compressor **5** that raises the pressure to 50 bar. The pressurized recycle was then mixed via a mixer **6** with the fresh CO₂ feed stream (15,919 kg/hr, 20°C, 100 bar, containing 50 ppm water) and the two were fed together to a feed effluent heat exchanger (FEHE) **7** and a start-up pre-heater **8** to raise the temperature to 225°C at the reactor inlet. The reactor was a multi-tubular plug-flow reactor **9** operated practically isothermally at 50 bar and 250°C, filled with solid catalyst (1.2% of the feed flow rate expressed in kg/hr). The catalyst and kinetics are described in the papers of:
1. Hye-Won Lim, Myung-June Park, Suk-Hwan Kang, Ho-Jeong Chae, Jong Wook Bae and Ki-Won Jun, 'Modeling of the Kinetics for Methanol Synthesis using Cu/ZnO/Al2O3/ZrO2 Catalyst: Influence of Carbon Dioxide during Hydrogenation', Ind. Eng. Chem. Res. Vol 48, 10448-10455 (2009).
2. AN Xin, ZUO Yizan, ZHANG Qiang and WANG Jinfu, 'Methanol Synthesis from CO2 Hydrogenation with a Cu/Zn/Al/Zr Fibrous Catalyst', Chinese Journal of Chemical Engineering, Vol 17(1), 88-94 (2009).

The reactants molar ratio H₂ : COₓ (with COₓ being CO₂ + CO) was 6 : 1 (the molar ratio H₂ : CO₂ was 6.85 : 1) and the CO₂ conversion per pass was over 31%. The flow rate of gas through the reactor is 72,380 kg/hr, with a volumetric composition of the reactor inlet gas (85.3% H₂, 12.5% CO₂, and 1.7% CO) that changed to 4.7% MeOH, 4.2% H₂O, 79.8% H₂, 9.2% CO₂, and 1.9% CO at the reactor outlet. The outlet stream was fed to the FEHE unit **7**, to pre-heat the reactor inlet using the heat of the reactor outlet stream, and then cooled down to 30°C via a cooler **10** and fed to the flash separation unit **3**, as mentioned before. Note that the heat produced in the reactor was used to generate high-pressure steam (over 4 MW energy), usable in the reboiler of the MeOH-H₂O distillation column **11** - thus covering ∼60% of the heating used by distillation.
The bottom outlet of the stripping column **2** (i.e. the purified methanol product stream) contained MeOH (63.1 %wt) and water (36.9 %wt) and only traces of CO₂ (470 ppm). This clearly shows the double positive effect of the stripping: the removal of practically all CO₂ and CO from the liquid phase by transferring them to the gas phase that is recycled and the complete removal of water from the gas phase (hydrogen) by transferring it to the liquid phase sent to distillation.
This bottom stream of the stripping column **2** - a mixture of methanol and water - was then separated in a single distillation column with a partial condenser **11**, resulting in water as bottom product (6,795 kg/hr) and lights as vapour distillate (11.6 kg/hr) and very high purity methanol (<100 ppm water and lights) as liquid distillate (11,568 kg/hr).
The overall methanol yield was 99.99%, with raw materials consumption figures close to the stoichiometric values: 1.376 kg CO₂ and 0.189 kg H₂ per ton of methanol product. The specific energy use was 550 kW.h/ton methanol (electricity) and 1.2 ton steam/ton methanol product (or 0.5 ton steam/ton methanol, taking into account the steam generated by the reactor).
It is noted that Figure 1 comprises a splitter **12** in case part of the first recycle gas stream needs to be purged. This, however, is optional. In the Example, there is no purge of the first recycle stream.

### Example 2

For this Example, we considered the same process as described in Example 1 but with the plug flow reactor **9** being operated at a lower temperature of 225°C instead of 250°C. The composition of the key process streams is the following. The counter-current feed stream fed to the top of the stripping column **2** is the methanol and water-rich liquid stream (18,881 kg/hr, 62.2 %wt MeOH, 34.5 %wt H₂O, 3.2 %wt CO₂, and 13 ppm CO, i.e. methanol-comprising product stream) from the flash separation unit **3**. The liquid stream from the stripping column partial condenser **4** was returned to the stripping column **2**, whereas the gas stream, consisting of H₂ (73.7 %wt), CO₂ (20.6 %wt), MeOH (5.6 %wt), CO (82 ppm), and water (125 ppm), was subsequently sent back to the flash separation unit **3**.

The top gas outlet of the stripping column partial condenser **4** was fed, together with the cooled reactor outlet stream comprising H₂ (19.2 %wt), CO₂ (59.3 %wt), MeOH (13.5 %wt), CO (1.5 %wt), and water (6.5 %wt), to the flash separation unit **3** operated at 45 bar and 30°C. The liquid outlet of the flash separation unit **3** was fed to the top of the stripping column **2** (as previously mentioned), while the vapour/gas outlet (i.e. the first recycle gas stream) was fed to a recycle compressor **5** that raises the pressure to 50 bar. The pressurized recycle was then mixed in mixer **6** with the fresh CO₂ feed stream (15,919 kg/hr, 20°C, 100 bar, containing 50 ppm water) and the two were fed together to a feed effluent heat exchanger (FEHE) **7**, a start-up pre-heater **8,** and a multi-tubular plug-flow reactor **9** operated at 50 bar and 225°C. The reactants molar ratio H₂ : COₓ (with COₓ being CO₂ + CO) was 6:1 (the molar ratio H₂ : CO₂ was 6.22 : 1) and the CO₂ conversion per pass was over 21% (due to a lower operating temperature in the reactor and hence lower reaction rates). The flow rate of gas through the reactor **9** is 100,239 kg/hr, with a volumetric composition of the reactor inlet gas (85.3% H₂, 13.7% CO₂, and 0.4% CO) that changed to 3.6% MeOH, 3.1% H₂O, 81.4% H₂, 11.5% CO₂, and 0.5% CO at the reactor outlet.

The bottom outlet of the stripping column **2** (i.e. the purified methanol product stream) contained MeOH (63.1 %wt) and water (36.9 %wt) and only traces of CO₂ (592 ppm). This clearly shows the double positive effect of the stripping: the removal of practically all CO₂ and CO from the liquid phase and all the water from the gas phase. The bottom stream of the stripping column **2** - a mixture of methanol and water - was then separated in a single distillation column with a partial condenser **11**, resulting in water as bottom product (6,794 kg/hr) and lights as vapour distillate (11.6 kg/hr) and very high purity methanol (<100 ppm water and lights) as liquid distillate (11,566 kg/hr).

The overall methanol yield was 99.99%, with raw materials consumption figures close to the stoichiometric values: 1.376 kg CO₂ and 0.189 kg H₂ per ton of methanol product. The specific energy use was 588 kW.h/ton methanol (electricity) and 1.52 ton steam/ton methanol product (or 0.82 ton steam/ton methanol taking into account the steam generated by the reactor).

### Comparative Example A

For the same CO₂ feed flow rate as in Example 2, we considered a process in which the stoichiometric wet hydrogen feed stream was pressurized, while the CO₂ feed stream was depressurized and heated, then mixed together in mixer with the recycle gas stream from flash separation unit and precompressed by recycle compressor and subsequently fed via a pre-heat exchanger to a plug flow reactor operated at the same conditions as described in Example 2. The rest of the process flow sheet was similar, but the key difference was the absence of the stripping column and stripping column condenser. It was found that CO₂ remained dissolved in the methanol-water stream (18,279 kg/hr, comprising 60.8 %wt methanol, 35.6 %wt water, 3.5 %wt CO₂, and 140 ppm CO). Hence, a direct sequence of two distillation columns was needed in the process - one to remove the significant amount of lights (606 kg/hr), including substantial amounts of CO, and the other to separate methanol (11,138 kg/hr) from water (6,533 kg/hr), in order to obtain methanol with the same purity as obtained via the process of Example 2.

In the process according to Example 2, the wet hydrogen feed stream was used to strip the CO₂ out of the condensable stream from the flash separation unit, leading to the complete recovery of the CO₂ dissolved in that stream - followed by its recycle to the reactor via the recycle compressor. The complete recovery of CO₂ allowed complete conversion (no CO₂ loss), the distillation of the methanol-water mixture in only one distillation column, and improved consumption figures.

Table 1 provides a brief comparison of the key performance indicators. Significant energy savings of 15% and 5% increased MeOH yield have been achieved using the process of Example 2 as compared to the process of Comparative Example A.

**Table 1**

| **Key performance indicators** | **Comparative Example A** | **Example 2** |
|---|---|---|
| CO₂ conversion per pass (%) | 20.90 | 21.51 |
| CO₂ conversion overall (%) | 95.18 | 99.78 |
| Total MeOH production rate (kg/hr) | 11,032 | 11,565 |
| Electricity consumption (kW.h/ton MeOH) | 607 | 588 |
| Steam consumption (ton steam/ton MeOH) | 1.92 | 1.52 |
| CO₂ use per unit of methanol product (kg/kg) | 1.494 | 1.376 |
| H₂ usage per unit of methanol product (kg/kg) | 0.197 | 0.189 |

### Example 3

For this Example, the process as described in Example 2 was employed, this time with a dry hydrogen feed being used. The composition of the key process streams is the following. The counter-current feed stream fed to the top of the stripping column was the methanol and water-rich liquid stream (18,905 kg/hr, 62.2 %wt MeOH, 34.5 %wt H₂O, 3.4 %wt CO₂, and 13 ppm CO, i.e. the methanol-comprising product stream) from the flash separation unit. The liquid stream from the stripping column partial condenser **4** was returned to the stripping column, whereas the gas stream, which consisted of H₂ (73.2 %wt), CO₂ (21.3 %wt), MeOH (5.6 %wt), CO (83 ppm), and water (125 ppm), was subsequently sent back to the flash separation unit and.

The top gas outlet of the stripping column partial condenser **4** was fed, together with the cooled reactor outlet stream comprising H₂ (18.6 %wt), CO₂ (60.2 %wt), MeOH (13.3 %wt), CO (1.5 %wt), and water (6.4 %wt), to the flash separation unit operated at 45 bar and 30°C. The liquid outlet of the flash unit was fed to the top of the stripping column, while the vapour/gas outlet (i.e. the first recycle gas stream) was fed to a recycle compressor that raised the pressure to 50 bar. The pressurized recycle was then mixed with the fresh CO₂ feed stream (15,919 kg/hr, 20 °C, 100 bar, containing 50 ppm water) and the two were fed together to a feed effluent heat exchanger (FEHE), a start-up pre-heater, and a multi-tubular plug-flow reactor operated at 50 bar and 225°C. The reactants molar ratio H₂ : COₓ was ∼6 : 1 (the molar ratio H₂ : CO₂ was 6.00 : 1) and the CO₂ conversion per pass was over 21%. The flow rate of gas through the reactor was 101,632 kg/hr, with a volumetric composition of the reactor inlet gas (85.0% H₂, 14.2% CO₂, and 0.4% CO) that changed to 3.6% MeOH, 3.1% H₂O, 80.9% H₂, 11.9% CO₂, and 0.5% CO at the reactor outlet.

The bottom outlet of the stripping column (i.e. the purified methanol product stream) contained MeOH (64.0 %wt) and water (36.0 %wt) and only traces of CO₂ (648 ppm). This proves again the positive effect of the stripping: the removal of practically all CO₂ and CO from the liquid phase. The bottom stream of the stripping column was then separated in a single distillation column with a partial condenser, resulting in water as bottom product (6,521 kg/hr) and lights as vapour distillate (11.6 kg/hr) and very high purity methanol (<100 ppm water and lights) as liquid distillate (11,568 kg/hr).

The overall methanol yield was 99.99%, with raw materials consumption figures close to the stoichiometric values: 1.376 kg CO₂ and 0.189 kg H₂ per ton of methanol product. The specific energy use was 588 kW.h/ton methanol (electricity) and 1.52 ton steam/ton methanol product.

### Comparative Example B

For the same CO₂ feed flow rate as in Example 3, we considered a process in which the stoichiometric dry hydrogen feed stream is pressurized, while the CO₂ feed stream is depressurized and heated, then mixed together with the recycle gas stream and subsequently fed via a pre-heat exchanger to a plug flow reactor operated at the same conditions as described in Example 3. The rest of the process flow sheet was similar, but the key difference was the absence of the stripping column with the stripping column partial condenser. It was found that CO₂ and CO remained dissolved in the methanol-water stream (18,020 kg/hr, comprising 61.7 %wt methanol, 34.7 %wt water, 3.5 %wt CO₂, and 144 ppm CO). Hence, a direct sequence of two distillation columns was needed in the process - one to remove the significant amount of lights (606 kg/hr), and the other to separate methanol (10,688 kg/hr) from water (6,725 kg/hr), in order to obtain methanol with the same purity as obtained via the process of Example 3. In the process according to Example 3, the hydrogen feed stream was used to strip the CO₂ out of the condensable stream from the flash separation unit, leading to the complete recovery of the CO₂ dissolved in that stream - followed by its recycle to the reactor via the recycle compressor. The complete recovery of CO₂ allowed complete conversion (no CO₂ loss and no CO emissions), the distillation of the methanol-water mixture in only one distillation column, and improved consumption figures.

Table 2 provides a brief comparison of the key performance indicators. Significant energy savings of 20% and 8% increased MeOH productivity have been achieved using the process of Example 3 as compared to the process of Comparative Example B.

**Table 2**

| **Key performance indicators** | **Comparative Example B** | **Example 3** |
|---|---|---|
| CO₂ conversion per pass (%) | 21.50 | 21.55 |
| CO₂ conversion overall (%) | 95.18 | 99.78 |
| Total MeOH production rate (kg/hr) | 10,657 | 11,567 |
| Electricity consumption (kW.h/ton MeOH) | 597 | 581 |
| Steam consumption (ton steam/ton MeOH) | 1.77 | 1.52 |
| CO₂ use per unit of methanol product (kg/kg) | 1.443 | 1.376 |
| H₂ usage per unit of methanol product (kg/kg) | 0.190 | 0.189 |

## Claims

1. A continuous process for the preparation of methanol by hydrogenation of carbon dioxide in the presence of a solid catalyst comprising the steps of
(i) feeding:
- (a) a feed comprising carbon dioxide;
- optionally, (b) a hydrogen feed;
- (c) at least part of a first recycle gas stream comprising carbon dioxide and hydrogen; and
- optionally, (d) at least part of a second recycle gas stream comprising carbon dioxide, carbon monoxide, and hydrogen,
to a reactor, to obtain a gaseous feed with a hydrogen: carbon dioxide molar ratio of between 2-18 : 1;
(ii) in said reactor, contacting said gaseous feed with a catalyst at a temperature of between 200 and 300°C and a pressure of between 40 and 200 bar, thereby forming an outlet stream comprising methanol, water, carbon monoxide, carbon dioxide, and hydrogen;
(iii) cooling the outlet stream;
(iv) subjecting said cooled outlet stream to a separation step, while optionally at least part of a second recycle gas stream is added to said outlet stream comprising methanol prior to and/or during said separation step, in which separation step methanol and water are separated from non-condensable components, thereby forming a methanol-comprising product stream and a first recycle gas stream;
(v) stripping the methanol-comprising product stream using a hydrogen stream, thereby forming a purified methanol product stream and a second recycle gas stream comprising carbon dioxide, carbon monoxide, and hydrogen; and
(vi) feeding at least part of the first recycle gas stream to step (i) and at least part of the second recycle gas stream to steps (i) and/or (iv).

2. Process according to claim 1, wherein the reactor of steps (i) and (ii) is a reactor selected from the group consisting of a multi-tubular plug-flow reactor, a fixed bed reactor, and a fluidized bed reactor.

3. Process according to claim 1 or 2, wherein step (v): is performed in a stripping column with trays or packing internals or in a flash vessel:

4. Process according to any one of the preceding claims, wherein the hydrogen stream of step (v) and/or the hydrogen feed of step (i) are hydrogen sources obtained from a chlorine production plant or a sodium chlorate production plant.

5. Process according to any one of the preceding claims, wherein the catalyst is a Cu/ZnO-based catalyst.

6. Process according to any one of the preceding claims, wherein the combined gaseous stream obtained in step (i) is heated in a feed-effluent heat-exchanger by the outlet stream obtained in step (ii) prior to being subjected it to step (ii).

7. Process according to any one of the preceding claims, wherein the carbon dioxide feed is depressurized prior to step (i), the hydrogen feed is pressurized to 30 - 50 bar prior to step (i), and wherein the combined gaseous feed is pressurized to 50 - 200 bar prior to step (ii).

8. Process according to any one of the preceding claims, wherein the feed comprising carbon dioxide, optionally a hydrogen feed, a first recycle gas stream comprising carbon dioxide and hydrogen, and a second recycle gas stream comprising carbon dioxide, hydrogen, and carbon monoxide, is fed to a reactor as one combined gaseous feed.

9. Process according to any one of the preceding claims, wherein in a step (vii), the methanol-comprising product stream is concentrated and/or purified.

10. Process according to any one of claims 2-9, wherein the combined gaseous feed is fed along the reactor at different stages.

11. Process according to any one of the preceding claims, wherein step (iii) is carried out in a feed-effluent heat-exchanger and, optionally, subsequently in a reboiler of a stripping column.

12. Process according to any one of the preceding claims, wherein the feed comprising carbon dioxide is carbon dioxide obtained from a bioethanol fermentation process and pressurized, such as is available from carbon capture and storage activities.

13. Process according to any one of the preceding claims, wherein in step (vi) the entire second recycle gas stream is fed to step (i).

14. Process according to any one of the preceding claims, wherein the hydrogen used in step (i) and/or the hydrogen stream of step (v) is a wet hydrogen source.

15. Process according to any one of the preceding claims wherein step (ii) of the process is carried out in two reactors instead of in one, with intermediate removal of water formed in the first reactor.

## Patentansprüche

1. Kontinuierliches Verfahren zur Herstellung von Methanol durch Hydrierung von Kohlendioxid in Gegenwart eines festen Katalysators, umfassend die folgenden Schritte:
(i) Zuführen:
- (a) einer Zufuhr mit Kohlendioxid;
- wahlweise (b) einer Wasserstoffzufuhr;
- (c) mindestens eines Teils eines ersten Recycle-Gasstroms mit Kohlendioxid und Wasserstoff; und
- wahlweise (d) mindestens eines Teils eines zweiten Recycle-Gasstroms mit Kohlendioxid, Kohlenmonoxid und Wasserstoff, zu einem Reaktor, um eine gasförmige Zufuhr mit einem Wasserstoff : Kohlendioxid-Molverhältnis von zwischen 2-18 : 1 zu erhalten;
(ii) in dem Reaktor: Inkontaktbringen der gasförmigen Zufuhr mit einem Katalysator bei einer Temperatur von zwischen 200 und 300°C und einem Druck von zwischen 40 und 200 bar, wobei ein Auslassstrom mit Methanol, Wasser, Kohlenmonoxid und Wasserstoff gebildet wird;
(iii) Abkühlenlassen des Auslassstromes;
(iv) Unterwerfen des gekühlten Auslassstromes unter einen Trennungsschritt, während vor und/oder während des Trennungsschrittes wahlweise mindestens ein Teil eines zweiten Recycle-Gasstromes dem Auslassstrom mit Methanol hinzugefügt wird, wobei in dem Trennungsschritt Methanol und Wasser von nicht kondensierbaren Komponenten getrennt werden, wodurch ein Methanol umfassender Produktstrom und ein erster Recycle-Gasstrom gebildet werden;
(v) Stripping des Methanol umfassenden Produktstromes unter Verwendung eines Wasserstoffstromes, wodurch ein gereinigter Methanolproduktstrom und ein zweiter Recycle-Gasstrom mit Kohlendioxid, Kohlenmonoxid und Wasserstoff gebildet werden; und
(vi) Zuführen von mindestens einem Teil des ersten Recycle-Gasstromes zu Schritt (i) und mindestens einem Teil des zweiten Recycle-Gasstromes zu den Schritten (i) und/oder (iv).

2. Verfahren nach Anspruch 1, wobei der Reaktor aus den Schritten (i) und (ii) ein Reaktor ist, ausgewählt aus der Gruppe, bestehend aus einem Multirohr-Kolbenstromreaktor, einem Festbett-Reaktor und einem Wirbelschichtreaktor.

3. Verfahren nach Anspruch 1 oder 2, wobei der Schritt (v) in einer Strippkolonne mit Böden oder Packungseinbauten oder in einem Flash-Behälter durchgeführt wird.

4. Verfahren nach einem der vorhergehenden Ansprüche, wobei der Wasserstoffstrom aus Schritt (v) und/oder die Wasserstoffzufuhr aus Schritt (i) Wasserstoffquellen sind, die man aus einer Chlor-Herstellungsanlage oder einer Natriumchlorat-Herstellungsanlage erhalten hat.

5. Verfahren nach einem der vorhergehenden Ansprüche, wobei der Katalysator ein Cu/ZnO-basierter Katalysator ist.

6. Verfahren nach einem der vorhergehenden Ansprüche, wobei der kombinierte gasförmige Strom, den man in Schritt (i) erhalten hat, in einem Zufuhr-Abfluss-Wärmetauscher durch den Auslassstrom erhitzt wird, den man in Schritt (ii) erhalten hat, bevor er Schritt (ii) unterworfen wird.

7. Verfahren nach einem der vorhergehenden Ansprüche, wobei der Druck bei der Kohlendioxidzufuhr vor Schritt (i) herabgesetzt wird, die Wasserstoffzufuhr vor Schritt (i) unter einen Druck von 30 - 50 bar ge-setzt wird, und wobei die kombinierte gasförmige Zufuhr vor Schritt (ii) unter einen Druck von 50 - 200 bar gesetzt wird.

8. Verfahren nach einem der vorhergehenden Ansprüche, wobei die Zufuhr mit Kohlendioxid, wahlweise eine Wasserstoffzufuhr, ein erster Recycle-Gasstrom mit Kohlendioxid und Wasserstoff, und ein zweiter Recycle-Gasstrom mit Kohlendioxid, Wasserstoff und Kohlenmonoxid als eine kombinierte gasförmige Zufuhr einem Reaktor zugeführt werden.

9. Verfahren nach einem der vorhergehenden Ansprüche, wobei in einem Schritt (vii) der Methanol umfassende Produktstrom konzentriert und/ oder gereinigt ist.

10. Verfahren nach einem der Ansprüche 2-9, wobei die kombinierte gasförmige Zufuhr entlang dem Reaktor in verschiedenen Stufen zugeführt wird.

11. Verfahren nach einem der vorhergehenden Ansprüche, wobei Schritt (iii) in einem Zufuhr-Abfluss-Wärmetauscher und wahlweise anschließend in einem Reboiler einer Strippkolonne durchgeführt wird.

12. Verfahren nach einem der vorhergehenden Ansprüche, wobei die Zufuhr mit Kohlendioxid ein Kohlendioxid ist, das man aus einem Bioethanol-Fermentationsprozess erhalten hat, und das unter Druck gesetzt wurde, wie es aus Kohlenstoffabscheidung und -speicherungsaktivitäten verfügbar ist.

13. Verfahren nach einem der vorhergehenden Ansprüche, wobei in Schritt (vi) der gesamte zweite Recycle-Gasstrom Schritt (i) zugeführt wird.

14. Verfahren nach einem der vorhergehenden Ansprüche, wobei der in Schritt (i) verwendete Wasserstoff und/oder der Wasserstoffstrom aus Schritt (v) eine nasse Wasserstoffquelle ist.

15. Verfahren nach einem der vorhergehenden Ansprüche, wobei Schritt (ii) des Verfahrens in zwei Reaktoren anstatt in einem durchgeführt wird, mit zwischenzeitlicher Entfernung von Wasser, das in dem ersten Reaktor gebildet wird.

## Revendications

1. Procédé en continu pour la préparation de méthanol par hydrogénation du dioxyde de carbone en présence d'un catalyseur solide comprenant les étapes suivantes :
(i) introduire :
- (a) une charge comprenant du dioxyde de carbone ;
- facultativement, (b) une charge d'hydrogène ;
- (c) au moins une partie d'un premier flux de gaz de recyclage comprenant du dioxyde de carbone et de l'hydrogène ; et
- facultativement, (d) au moins une partie d'un deuxième flux de gaz de recyclage comprenant du dioxyde de carbone, du monoxyde de carbone et de l'hydrogène,
dans un réacteur, pour obtenir une charge gazeuse avec un rapport molaire hydrogène : dioxyde de carbone compris entre 2 et 18 : 1 ;
(ii) mettre en contact, dans ledit réacteur, ladite charge gazeuse avec un catalyseur à une température comprise entre 200 et 300°C et une pression comprise entre 40 et 200 bars, formant ainsi un flux de sortie comprenant du méthanol, de l'eau, du monoxyde de carbone, du dioxyde de carbone et de l'hydrogène ;
(iii) refroidir le flux de sortie ;
(iv) soumettre ledit flux de sortie refroidi à une étape de séparation, tandis que, facultativement, au moins une partie d'un deuxième flux de gaz de recyclage est ajoutée audit flux de sortie comprenant du méthanol avant et/ou pendant ladite étape de séparation, dans laquelle étape de séparation, le méthanol et l'eau sont séparés des composants non condensables, formant ainsi un flux de produit comprenant du méthanol et un premier flux de gaz de recyclage ;
(v) entrainement du flux de produit comprenant du méthanol en utilisant un flux d'hydrogène, formant ainsi un flux de produit de méthanol purifié et un deuxième flux de gaz de recyclage comprenant du dioxyde de carbone, du monoxyde de carbone et de l'hydrogène ; et
(vi) introduire au moins une partie du premier flux de gaz de recyclage à l'étape (i) et au moins une partie du deuxième flux de gaz de recyclage aux étapes (i) et/ou (iv).

2. Procédé selon la revendication 1, dans lequel le réacteur des étapes (i) et (ii) est un réacteur choisi dans le groupe constitué d'un réacteur à écoulement piston multitubulaire, d'un réacteur à lit fixe et d'un réacteur à lit fluidisé.

3. Procédé selon la revendication 1 ou 2, dans lequel l'étape (v) est réalisée dans une colonne d'extraction avec des plateaux ou des éléments internes de garnissage ou dans un ballon de flashing.

4. Procédé selon l'une quelconque des revendications précédentes, dans lequel le flux d'hydrogène de l'étape (v) et/ou la charge d'hydrogène de l'étape (i) est/sont des sources d'hydrogène obtenu(e)(s) à partir d'une usine de production de chlore ou d'une usine de production de chlorate de sodium.

5. Procédé selon l'une quelconque des revendications précédentes, dans lequel le catalyseur est un catalyseur à base de Cu/ZnO.

6. Procédé selon l'une quelconque des revendications précédentes, dans lequel le flux gazeux combiné obtenu dans l'étape (i) est chauffé dans un échangeur de chaleur charge-effluent par le flux de sortie obtenu dans l'étape (ii) avant d'être soumis à l'étape (ii).

7. Procédé selon l'une quelconque des revendications précédentes, dans lequel la charge de dioxyde de carbone est dépressurisée avant l'étape (i), la charge d'hydrogène est pressurisée à une pression comprise entre 30 et 50 bars avant l'étape (i), et où la charge gazeuse combinée est pressurisée à une pression comprise entre 50 et 200 bars avant l'étape (ii).

8. Procédé selon l'une quelconque des revendications précédentes, dans lequel la charge comprenant du dioxyde de carbone, facultativement une charge d'hydrogène, un premier flux de gaz de recyclage comprenant du dioxyde de carbone et de l'hydrogène et un deuxième flux de gaz de recyclage comprenant du dioxyde de carbone, de l'hydrogène et du monoxyde de carbone, est introduite dans un réacteur en tant que charge gazeuse combinée.

9. Procédé selon l'une quelconque des revendications précédentes, dans lequel, dans l'étape (vii), le flux de produit comprenant du méthanol est concentré et/ou purifié.

10. Procédé selon l'une quelconque des revendications 2 à 9, dans lequel la charge gazeuse combinée est introduite le long du réacteur à différents étapes.

11. Procédé selon l'une quelconque des revendications précédentes, dans lequel l'étape (iii) est effectuée dans un échangeur de chaleur charge-effluent et, facultativement, par la suite, dans un rebouilleur d'une colonne d'extraction.

12. Procédé selon l'une quelconque des revendications précédentes, dans lequel la charge comprenant du dioxyde de carbone est le dioxyde de carbone obtenu à partir d'un procédé de fermentation en bioéthanol et pressurisée, telle que celle disponible dans les activités de capture et de stockage de carbone.

13. Procédé selon l'une quelconque des revendications précédentes, dans lequel, dans l'étape (vi) tout le deuxième flux de gaz de recyclage est introduit à l'étape (i).

14. Procédé selon l'une quelconque des revendications précédentes, dans lequel l'hydrogène utilisé dans l'étape (i) et/ou le flux d'hydrogène de l'étape (v) est/sont une source d'hydrogène humide.

15. Procédé selon l'une quelconque des revendications précédentes, dans lequel l'étape (ii) du procédé est effectuée dans deux réacteurs au lieu d'un, avec une élimination intermédiaire de l'eau formée dans le premier réacteur.
